(19) Europäisches Patentamt
European Patent Office
Office européen des brevets

(11) Veröffentlichungsnummer: 0 257 445 B1

(12) **EUROPÄISCHE PATENTSCHRIFT**

(45) Veröffentlichungstag der Patentschrift: **12.07.89**

(51) Int. Cl.⁴: **C07C 43/315**, C07C 41/48

(21) Anmeldenummer: **87111651.3**

(22) Anmeldetag: **12.08.87**

(54) **Verfahren zur Herstellung von 1,1,2-Trialkoxyethan.**

(30) Priorität: **16.08.86 DE 3627776**

(43) Veröffentlichungstag der Anmeldung: **02.03.88 Patentblatt 88/9**

(45) Bekanntmachung des Hinweises auf die Patenterteilung: **12.07.89 Patentblatt 89/28**

(84) Benannte Vertragsstaaten: **BE DE ES FR GB IT NL**

(56) Entgegenhaltungen:
**DE-A- 2 655 406**
**US-A- 2 449 470**

(73) Patentinhaber: **BASF Aktiengesellschaft, Carl-Bosch-Strasse 38, D-6700 Ludwigshafen(DE)**

(72) Erfinder: **Maerkl Robert, Dr., Schulstrasse 17, D-6701 Fussgoenheim(DE)**
Erfinder: **Bertleff, Werner, Dr., Neuzenlache 3, D-6806 Viernheim(DE)**
Erfinder: **Kummer, Rudolf, Dr., Kreuzstrasse 6, D-6710 Frankenthal(DE)**

**EP 0 257 445 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von 1,1,2-Trialkoxyethan.

1,1,2-Trialkoxyethan stellt ein vielseitig verwendbares Zwischenprodukt für organische Synthesen dar. Beispielsweise kann daraus durch Acetalspaltung Alkoxyacetaldehyd hergestellt werden, das durch Kondensation mit Formaldehyd zu Polyolen umgesetzt werden kann. Durch Eliminierung von Alkohol kann weiterhin Dialkoxyethen, z.B. Dimethoxyethen hergestellt werden, das als Ausgangsstoff für Polymerisate dient.

Gemäß der DE-OS 20 48 272 ist bekannt, daß 1,1,2-Trimethoxyethan durch Umsetzung von 1-Chlor-2,2-dimethoxyethan oder 1,1-Dichlor-2-methoxyethan mit Alkalimethylat gewonnen werden kann. Als Nebenprodukt entsteht jedoch zu 20 % ortho-Essigsäuremethylester. Ein weiterer Nachteil dieses Verfahrens liegt in dem unvermeidbaren Anfall von Alkalichlorid.

Der Erfindung lag nun die Aufgabe zugrunde, ein Verfahren zur Herstellung von 1,1,2-Trialkoxyethan zu finden, nach dem die Produkte in guten Ausbeuten ohne Anfall von Salzen erhalten werden.

Demgemäß wurde ein Verfahren zur Herstellung von 1,1,2-Trialkoxyethan der Formel I
i

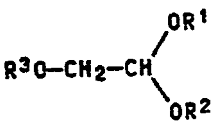

(I)

n der die Reste $R^1$ bis $R^3$ $C_1$-$C_8$-Alkylreste bedeuten, wobei die Reste $R^1$ und $R^2$ auch miteinander unter Ausbildung eines 5- bis 7-gliedrigen Ringes verknüpft sein können, gefunden, das dadurch gekennzeichnet ist, daß man Formaldehyddialkylacetale II mit Kohlenmonoxid und Wasserstoff, wobei pro Mol Kohlenmonoxid 0,5 bis 1,5 mol Wasserstoff verwendet werden und mit mindestens 1 mol Alkohol $R^3OH$ pro Mol Formaldehyddialkylacetal bei erhöhtem Druck und erhöhter Temperatur in Gegenwart eines Katalysators umsetzt, der aus einer Kobaltcarbonylverbindung und einer dreiwertigen organischen Verbindung der Formel III

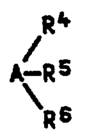

III,

in der A für Phosphor, Arsen, Antimon oder Wismut steht und in der $R^4$ bis $R^6$ organische Reste bedeuten, gebildet wird.

Der Erfolg des Verfahrens ist überraschend; denn gemäß der DE-OS 26 55 406 ist bekannt, daß durch Hydroformylierung von Formaldehyddialkylacetalen in Gegenwart eines Katalysators, gebildet z.B. aus Dikobaltoctacarbonyl und Trialkylphosphin und in Gegenwart eines inerten Lösungsmittels, z.B. Toluol, Ethylenglykol in einer Ausbeute von ca. 67 % anfällt. Die Bildung von Trialkoxyethan wird nicht beschrieben. Als inerte Lösungsmittel werden neben Ethern und aliphatischen oder aromatischen Kohlenwasserstoffen auch Alkohole aufgeführt. Das $CO/H_2$-Verhältnis soll für den Ablauf der Reaktion nicht entscheidend sein und kann 0,1 bis 10 mol $H_2$ pro Mol CO betragen. In den Beispielen wird stets ein hoher Überschuß an Wasserstoff verwendet.

Als Ausgangsverbindungen für das erfindungsgemäße Verfahren dienen Formaldehyddialkylacetale der Formel II

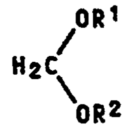

II,

in der die Reste $R^1$ und $R^2$ gleiche oder verschiedene $C_1$- bis $C_8$-Alkylreste bedeuten oder miteinander unter Ausbildung eines 5- bis 7-gliedrigen Ringes verknüpft sind. Vorzugsweise stehen $R^1$ und $R^2$ für primäre oder sekundäre $C_1$- bis $C_4$-Alkylreste, insbesondere für Methylreste. Beispielsweise seien der Methyl-, Ethyl-, Propyl-, Isopropyl, Butyl- oder Isobutylrest genannt. Cyclische Acetale sind z.B. 1,3-Dioxolan oder 1,3-Dioxan. Anstelle der Acetale II können auch deren Vorläufer, nämlich Formaldehyd oder Verbindungen, die unter den Reaktionsbedingungen Formaldehyd freisetzen, wie Paraformaldehyd oder Trioxan, und die entsprechenden Alkohole eingesetzt werden, wobei das Verhältnis von Aldehyd zu Alkohol nicht besonders kritisch ist. Zweckmäßigerweise wird man pro Mol Aldehyd 1 bis 5 mol Alkohol verwenden. Besonders bevorzugt werden Acetale umgesetzt, deren Alkoholkomponente dem für die Umsetzung verwendeten Alkohol $R^3OH$ entsprechen.

Als Alkohole $R^3OH$ dienen $C_1$- bis $C_8$-Alkohole, vorzugsweise $C_1$- bis $C_4$-Alkohole, insbesondere Methanol. Beispielsweise seien folgende Alkohole aufgeführt: Methanol, Ethanol, Propanol, Isopropanol, n-Butanol, iso-Butanol, tert.-Butanol, n-Pentanol, Isoamylalkohol, Neopentylalkohol, n-Hexanol, Hexanol-(2), n-Heptanol und n-Oktanol.

Das Formaldehyddialkylacetal II wird mit mindestens äquimolaren Mengen Alkohol $R^3OH$ umgesetzt. Vorteilhaft kann man den Alkohol im Überschuß einsetzen, z.B. 1 bis 5, vorzugsweise 1,1 bis 4, insbesondere 1,5 bis 2,5 mol Alkohol $R^3OH$ pro Mol Acetal. Höhere Überschüsse sind möglich, bringen aber keine weiteren Vorteile.

Für die Hydroformylierung der Acetale II wird ein Kohlenmonoxid-Wasserstoffgemisch verwendet, in dem pro Mol Kohlenmonoxid 0,5 bis 1,5 mol, insbesondere 0,5 bis 1 mol Wasserstoff vorliegen. Vorzugsweise liegt das Molverhältnis $CO:H_2$ bei 1:1.

Das erfindungsgemäße Verfahren wird in Gegenwart eines Komplexkatalysators durchgeführt, der aus einer Kobaltcarbonylverbindung und einer dreiwertigen organischen Verbindung III

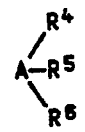 III,

in der A für Phosphor, Arsen, Antimon oder Wismut steht und die Reste $R^4$ bis $R^6$ organische Reste, z.B. Alkyl-, Cycloalkyl-, Alkoxy-, Aryl- oder Aryloxyreste bedeuten, gebildet wird.

Als Kobaltcarbonylverbindung können z.B. Dikobaltoctacarbonyl oder $HCo(CO)_4$ dienen. Die Kobaltcarbonylverbindungen können auch in situ aus Kobaltverbindungen, die unter den Reaktionsbedingungen Kobaltcarbonylkomplexe bilden können wie Kobaltsalze organischer oder anorganischer Säuren, z.B. Kobaltacetat, Kobaltlaurat, Kobaltnitrat, Kobaltsulfat oder Kobalthalogeniden oder aus Kobaltoxid hergestellt werden.

Als Verbindungen III werden vorzugsweise schwer flüchtige Triorganophosphorverbindungen verwendet, wobei Trialkyl- und Triarylphosphine sowie Trialkyl- und Triarylphosphite wirtschaftlich besonders interessant sind. Bevorzugt werden Triarylphosphine wie Triphenylphosphin oder Tritolylphosphin und insbesondere Trialkylphosphine, in denen die Reste $R^4$ bis $R^6$ eine $C_1$- bis $C_8$-Alkylgruppe bedeuten, z.B. Tributyl-, Triisopropyl- oder Trioctylphosphin. Als Reste $R^4$ bis $R^6$ können auch $C_5$- bis $C_7$-Cycloalkylreste, z.B. Cyclohexylreste dienen. Anstelle der genannten Phosphine können auch die entsprechenden tertiären Phosphite wie Triphenyl- oder Tributylphosphit verwendet werden.

Hervorzuheben sind auch preiswerte Alkylarylphosphine wie Diphenyl/$C_1$-$C_8$-Alkylphosphine oder $C_1$- bis $C_8$-Dialkyl/Phenylphosphine.

Vorteilhaft wird der Katalysator in situ aus der Kobaltcarbonylverbindung bzw. dessen Vorläufern und Verbindung III hergestellt. Es ist aber auch möglich, die Komplexverbindungen wie $HCo(CO)_3(PR^4,R^5,R^6)$ oder $Co_2(CO)_6(PR^4,R^5,R^6)$ usw. getrennt in üblicher Weise herzustellen und dann dem Reaktionsgemisch zuzusetzen.

Das Atomverhältnis von Phosphor zu Kobalt liegt im allgemeinen bei 0,1 bis 1,2, vorzugsweise 0,3 bis 0,9.

Bezogen auf das Formaldehyddialkylacetal II können vorteilhaft Kobaltkonzentrationen und Phosphinkonzentrationen zwischen 1 und 5 Mol.% gewählt werden. Höhere Konzentrationen sind möglich, aber wirtschaftlich wenig interessant.

Das erfindungsgemäße Verfahren kann in Gegenwart oder in Abwesenheit von Lösungsmitteln erfolgen. Als Lösungsmittel sind z.B. Ether wie Diethyl- oder Diphenylether, aromatische oder aliphatische Kohlenwasserstoffe wie Benzol, Toluol oder Hexan und Alkohole geeignet. Vorteilhaft dienen die für die Umsetzung benötigten Alkohole $R^3OH$ zugleich als Lösungsmittel.

Die Umsetzung kann diskontinuierlich oder vorzugsweise kontinuierlich bei einem Druck von 100 bis 700, insbesondere 200 bis 400 bar und einer Temperatur von 50 bis 300, vorzugsweise 100 bis 250°C nach den dafür üblichen Techniken durchgeführt werden.

Nach der Umsetzung können die Trialkoxyethane I in an sich bekannter Weise, z.B. durch Destillation aus dem Gemisch isoliert werden. Der Katalysator verbleibt im Destillationsrückstand und kann für das erfindungsgemäße Verfahren erneut wiederverwendet werden.

Beispiel 1

In einem Autoklaven wurden 645 g (8,50 mol) Formaldehyddimethylacetal und 272 g (18,5 mol) Methanol in Gegenwart von 24,2 g (0,07 mol) Dikobaltoctacarbonyl und 25,1 g (0,09 mol) Tributylphosphin bei 150°C und 280 bar innerhalb von 2 Stunden hydroformyliert, wobei das Verhältnis von CO zu $H_2$ 1:1 betrug. Der Austrag enthielt 252 g (3,35 mol) Formaldehyddimethylacetal und 372 g (3,1 mol) Trimethoxyethan, entsprechend einer Ausbeute von 60 %, bezogen auf umgesetztes Formaldehyddimethylacetal.

Beispiel 2

Analog Beispiel 1 wurden 645 g (8,50 mol) Formaldehyddimethylacetal, 272 g Methanol, 24,2 g (0,07 mol) Dicobaltoctacarbonyl und 25,5 g (0,09 mmol) Hexyldiphenylphosphin bei 150°C und 280 bar zur Reaktion gebracht. Der Umsatz an Formaldehyddimethylacetal betrug 47 % (4,0 mol), die Selektivität zu Trimethoxiethan 70 %.

**Patentansprüche**

1. Verfahren zur Herstellung von 1,1,2-Trialkoxyethan der Formel I

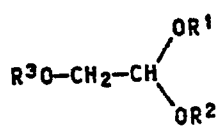 (I)

in der die Reste $R^1$ bis $R^3$ $C_1$-$C_8$-Alkylreste bedeuten, wobei die Reste $R^1$ und $R^2$ auch miteinander unter Ausbildung eines 5- bis 7-gliedrigen Ringes verknüpft sein können, dadurch gekennzeichnet, daß man Formaldehyddialkylacetale der Formel II

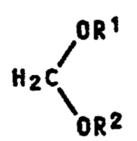 II,

in der die Reste $R^1$ $R^2$ gleiche oder verschiedene $C_1$- bis $C_8$-Alkylreste bedeuten oder miteinander unter Ausbildung eines 5- bis 7-gliedrigen Ringes verknüpft sind, mit Kohlenmonoxid und Wasserstoff, wobei pro Mol Kohlenmonoxid 0,5 bis 1,5 mol Wasserstoff verwendet werden, und mit mindestens 1 mol Alkohol $R^3OH$ pro Mol Formaldehyddialkylacetal bei erhöhtem Druck und erhöhter Temperatur in Gegenwart eines Katalysators umsetzt, der aus einer Kobaltcarbonylverbindung und einer dreiwertigen organischen Verbindung der Formel III

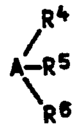

III,

in der A für Phosphor, Arsen, Antimon oder Wismut steht und in der $R^4$ bis $R^6$ organische Reste bedeuten, gebildet wird.

2. Verfahren nach Anspruch 1, dadurch gekennzeichnet, daß die Reste $R^1$ bis $R^3$ gleich sind.

3. Verfahren nach den Ansprüchen 1 und 2, dadurch gekennzeichnet, daß man pro Mol Formaldehyddialkylacetal 1 bis 5 mol Alkohol $R^3OH$ verwendet.

4. Verfahren nach den Ansprüchen 1 und 2, dadurch gekennzeichnet, daß man pro Mol Formaldehyddialkylacetal 1,5 bis 2,5 mol Alkohol $R^3OH$ verwendet.

5. Verfahren nach den Ansprüchen 1 bis 4, dadurch gekennzeichnet, daß man pro Mol Kohlenmonoxid 0,5 bis 1 mol Wasserstoff verwendet.

6. Verfahren nach den Ansprüchen 1 bis 5, dadurch gekennzeichnet, daß man äquimolare Mengen Kohlenmonoxid und Wasserstoff verwendet.

7. Verfahren nach den Ansprüchen 1 bis 6, dadurch gekennzeichnet, daß der Katalysator aus einer Kobaltcarbonylverbindung und einem tertiären Phosphin gebildet wird.

8. Verfahren nach den Ansprüchen 1 bis 7, dadurch gekennzeichnet, daß man als tertiäres Phosphin ein Trialkylphosphin verwendet.

9. Verfahren nach den Ansprüchen 1 bis 8, dadurch gekennzeichnet, daß man die Umsetzung bei einer Temperatur von 50 bis 300°C durchgeführt.

10. Verfahren nach den Ansprüchen 1 bis 9, dadurch gekennzeichnet, daß man die Umsetzung bei einem Druck von 100 bis 700 bar durchführt.

**Claims**

1. A process for the preparation of a 1,1,2-trialkoxyethane of the formula I

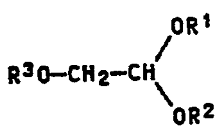

(I)

where $R^1$, $R^2$ and $R^3$ are each $C_1$-$C_8$-alkyl and $R^1$ and $R^2$ may furthermore be bonded to one another to form a 5-membered to 7-membered ring, wherein a formaldehyde dialkyl acetal of the formula II

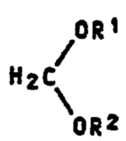

II

where $R^1$ and $R^2$ are identical or different $C_1$-$C_8$-alkyl or are bonded to one another to form a 5-membered, 6-membered or 7-membered ring, is reacted with carbon monoxide and hydrogen, from 0.5 to 1.5 moles of hydrogen being used per mole of carbon monoxide, and with not less than 1 mole of alcohol $R^3OH$ per mole of formaldehyde dialkyl acetal under superatmospheric pressure and at elevated temperatures in the presence of a catalyst which is formed from a cobalt carbonyl compound and a trivalent organic compound of the formula III

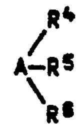

III

where A is phosphorus, arsenic, antimony or bismuth and $R^4$, $R^5$ and $R^6$ are each an organic radical.

2. A process as claimed in claim 1, wherein $R^1$, $R^2$ and $R^3$ are identical.

3. A process as claimed in either of claims 1 and 2, wherein from 1 to 5 moles of alcohol $R^3OH$ are used per mole of formaldehyde dialkyl acetal.

4. A process as claimed in either of claims 1 and 2, wherein from 1.5 to 2.5 moles of alcohol $R^3OH$ are used per mole of formaldehyde dialkyl acetal.

5. A process as claimed in any of claims 1 to 4, wherein from 0.5 to 1 mole of hydrogen is used per mole of carbon monoxide.

6. A process as claimed in any of claims 1 to 5, wherein equimolar amounts of carbon monoxide and hydrogen are used.

7. A process as claimed in any of claims 1 to 6, wherein the catalyst is formed from a cobalt carbonyl compound and a tertiary phosphine.

8. A process as claimed in any of claims 1 to 7, wherein the tertiary phosphine used is trialkylphosphine.

9. A process as claimed in any of claims 1 to 8, wherein the reaction is carried out at from 50 to 300°C.

10. A process as claimed in any of claims 1 to 9, wherein the reaction is carried out under from 100 to 700 bar.

**Revendications**

1. Procédé de préparation de 1,1,2-trialcoxyéthane de formule I

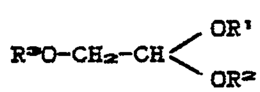

(I)

dans laquelle les radicaux $R^1$ à $R^3$ sont des restes alkyle en $C_1$ à $C_8$, les radicaux $R^1$ et $R^2$ pouvant être également reliés entre eux en formant un noyau penta- à heptagonal, caractérisé en ce qu'on fait réagir des dialkylacétals de formaldéhyde de formule II

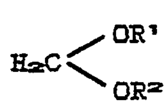

(II)

dans laquelle les radicaux $R^1$ et $R^2$ sont des restes alkyle en $C_1$ à $C_8$ identiques ou différents ou sont reliés entre eux en formant un noyau penta- à heptagonal, avec de l'oxyde de carbone et de l'hydrogène, en utilisant 0,5 à 1,5 mole d'hydrogène par mole

d'oxyde de carbone, et avec au moins 1 mole d'alcool $R^3OH$ par mole de dialkylacétal de formaldéhyde, sous pression élevée et à température élevée, en présence d'un catalyseur qui est formé à partir d'un composé carbonylé du cobalt et d'un composé organique trivalent de formule III

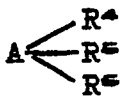

(III)

dans laquelle A est mis pour un atome de phosphore, d'arsenic, d'antimoine ou de bismuth et dans laquelle $R^4$ à $R^6$ représentent des restes organiques.

2. Procédé selon la revendication 1, caractérisé en ce que les radicaux $R^1$ à $R^3$ sont identiques.

3. Procédé selon la revendication 1 ou 2, caractérisé en ce qu'on utilise, par mole de dialkylacétal de formaldéhyde, 1 à 5 moles d'alcool $R^3OH$.

4. Procédé selon la revendication 1 ou 2, caractérisé en ce qu'on utilise, par mole de dialkylacétal de formaldéhyde, 1,5 à 2,5 moles d'alcool $R^3OH$.

5. Procédé selon l'une quelconque des revendications 1 à 4, caractérisé en ce qu'on utilise, par mole d'oxyde de carbone, 0,5 à 1 mole d'hydrogène.

6. Procédé selon l'une quelconque des revendications 1 à 5, caractérisé en ce qu'on utilise des quantités équimolaires d'oxyde de carbone et d'hydrogène.

7. Procédé selon l'une quelconque des revendications 1 à 6, caractérisé en ce que le catalyseur est formé à partir d'un composé carbonylé du cobalt et d'une phosphine tertiaire.

8. Procédé selon l'une quelconque des revendications 1 à 7, caractérisé en ce qu'on utilise, en tant que phosphine tertiaire, une trialkylphosphine.

9. Procédé selon l'une quelconque des revendications 1 à 8, caractérisé en ce qu'on conduit la réaction à une température de 50 à 300°C.

10. Procédé selon l'une quelconque des revendications 1 à 9, caractérisé en ce qu'on conduit la réaction sous une pression de 100 à 700 bars.